# EUROPEAN PATENT SPECIFICATION

(11) **EP 4 351 703 B1**
(45) Date of publication and mention of the grant of the patent: **02.12.2026**
(21) Application number: 22732266.6
(22) Date of filing: 10.06.2022
(51) Int. Cl.: A61N 1/05, A61N 1/372, A61N 1/375, A61B 5/374, A61B 5/377, A61N 1/36, A61B 5/053, A61B 5/00, A61B 5/263, A61B 5/293

(54) **SYSTEM FOR NEUROMODULATION APPLICATIONS**
SYSTEM FÜR NEUROMODULATIONSANWENDUNGEN
SYSTÈME POUR APPLICATIONS DE NEUROMODULATION

(30) Priority: 11.06.2021 EP 21382525
(43) Date of publication of application: 17.04.2024
(60) Divisional application: 26214148.4
(73) Proprietor: INBRAIN Neuroelectronics SL, 08028 Barcelona (ES)
(72) Inventor: BAKKER, Jurriaan, 5591PZ Heeze (NL); DONEGA, Matteo, 68330 Huningue (FR); RODRIQUEZ, Elisa, 47008 Valladolid (ES); VAN SCHUYLENBERGH, Koenraad F., 2290 Vorselaar (BE)
(74) Representative: K&L Gates LLP
(86) International application number: PCT/EP2022/065868
(87) International publication number: WO 2022/258820

(56) References cited:
- US-A1- 2014 180 361
- US-A1- 2019 224 474
- US-B2- 8 396 557
- NICHOLAS V. APOLLO ET AL: "Soft, Flexible Freestanding Neural Stimulation and Recording Electrodes Fabricated from Reduced Graphene Oxide", ADVANCED FUNCTIONAL MATERIALS, vol. 25, no. 23, 1 June 2015 (2015-06-01), Hoboken, USA, pages 3551 - 3559, XP055744108, ISSN: 1616-301X, DOI: 10.1002/adfm.201500110
- LU YICHEN ET AL: "Graphene-based neurotechnologies for advanced neural interfaces", vol. 6, 1 June 2018 (2018-06-01), pages 138 - 147, XP055892260, ISSN: 2468-4511, Retrieved from the Internet <URL:https://www.sciencedirect.com/science/article/pii/S2468451118300096/pdfft?md5=1140596a17f7a7a8e8be1fa0ff6067c2&pid=1-s2.0-S2468451118300096-main.pdf> DOI: 10.1016/j.cobme.2018.06.001

## Description

The present invention relates to a system for neuromodulation applications, in particular for monitoring and/or adaption of neuromodulation.

The present invention relates to the technical field of neuromodulation, for example to cortical and/or deep brain stimulation. Cortical and/or deep brain stimulation are methods for the diagnosis and therapy of neurodegenerative diseases such as *inter alia* the Parkinson's disease, epilepsy, and chronic pain. Electrical stimulation by means of leads which are implanted into brain areas or regions like the subthalamic nucleus and/or the globus pallidus internus can alleviate symptoms, such as tremor symptoms of a patient suffering from a drug-resistant Parkinson's disease. Further, the signals from a brain region or area at which the leads were implanted can be recorded and the state of the brain tissue can be determined using impedance measurements.

In this relation, neuroprosthetic devices are powerful tools to monitor, prevent and treat neural diseases, disorders and conditions by interfacing electrically with the nervous system. They are capable of recording and/or stimulating electrically neural activity once implanted in the nervous tissue. Currently, most neuroprosthetic technologies apply electrodes interfacing with neural tissue.

In more detail, in particular the vagus nerve, but also other peripheral neuromodulation and cranial neuromodulation tissues, e.g. spinal cord, afferent/efferent nerves to organs such as skin, intestines, lungs, kidneys, bladder, heart, sexual organs and the like, contain both efferent (parasympathetic and somatic motor) and afferent (sensory visceral and somatic sensory) nerve fibers involved in various neurophysiological controls.

Stimulating/Blocking/Modulating those nerves could have neurophysiological effects/responses on all of the respective functions.

Existing systems and methods, use time-consuming programming to find the optimum stimulation settings. However, those program protocols are limited to e.g. the time of the day, the history of drug intake, the patient's mood or the state of the patient.

Hence, the state of the art solely provides an optimum setting for specific circumstances, namely when and/or where the program protocol is applied, e.g. in a clinical environment. In contrast thereto, such specific program protocols may be sub-optimal under deviating conditions and circumstances, like the daily care at home.

In consequence, such existing systems may deliver stimulation to the patient at incorrect intensity, at incorrect intervals, with incorrect timing and/or even when the patient is not in need for any stimulation at all, due to the respective time of the day, developing effects in the course of drug intake/release and/or due to various (intended) activities of the patient during the day.

Not only may such deviations from an optimum therapy setting provide discomfort and health restrictions to the patient, but also influence the power consumption and the product life cycle of the system itself.

US8396557B2 discloses an intracranial stimulator adapted for bilateral implantation in a human patient, said system comprising two bilaterally-implanted neurological control systems each including a stimulating and recording unit and one or more intracranial components. In particular, an intracranial stimulation array comprising a plurality of intracranial stimulation electrodes, and an intracranial recording electrode array comprising a plurality of intracranial recording electrodes can be provided. Input signals from the intracranial stimulation array and the intracranial recording electrode array are transmitted to the stimulating and recording unit. The stimulating and recording unit is further adapted to receive ipsilateral, contralateral or bilateral inputs from different sensors, e.g., EMG sensor(s) or EEG sensor(s).

Further prior art is known from US 2019/224474 A1 and from Nicholas V. Apollo ET AL: "Soft, Flexible Freestanding Neural Stimulation and Recording Electrodes Fabricated from Reduced Graphene Oxide", Advanced Functional Materials, vol. 25, no. 23, 1 June 2015 (2015-06-01), pages 3551-3559.

It is an object of the invention to provide a system for neuromodulation which allows for monitoring and/or surveillance of the patient's condition, environment and/or circumstances in order to execute and adapt a neuromodulation therapy in a manner that allows to optimize the efficiency as well as the impact on the patient's health, whereby such optimization can be based on corresponding feedback data, in particular feedback data about the patient.

The aforementioned objects are solved by the subject-matters of the independent claim 1. Advantageous configurations of the invention are described in dependent claims.

According to the present invention a system for neuromodulation applications is provided, in particular for monitoring and/or adaption of neuromodulation, wherein the system comprises:
- at least one electrode device with at least one stimulation electrode and at least one recording electrode,
- at least one control unit, and
- at least one sensor unit for determining sensor data, in particular vital and/or non-vital parameters of the patient,
wherein the control unit is configured to adapt and/or initiate a neuromodulation, in particular an application of electrical pulses via the at least one stimulation electrode, on basis of current signals and/or voltage signals as determined by the at least one recording electrode and/or on basis of sensor data as provided by the at least one sensor unit.

The invention is based on the idea to gather and determine various kinds of information about the patient's condition and/or the patient's situation in order to individually adapt a neuromodulation therapy in appropriate manner at a specific time. Thus, the system according to the present invention is particularly configured to monitor and survey the patient in order to capture sufficient information on which basis the therapy can be adapted to the patient specific situation.

Preferably, the system as provided by the present invention can thus be considered in general as a closed-loop system, namely continuously or frequently gathering information for adaption of the ongoing treatment by controlling and/or regulating the therapy application, e.g. the application of stimulation pulses by the system.

The system may also be configured to be able to operate as an open loop system, in particular temporarily. In case of a partial system failure, namely if sensor data or the like should be missing e.g. due to a sensor failure, the system may be able to continue operation and its neuromodulation application. Further, the system may be configured to further adapt the continuing neuromodulation based on estimations and predictions about the patient's condition, in particular based on earlier data and as controlled/regulated by the control unit.

In particular, vital and/or non-vital parameter concerning the patient's situation can be taken into consideration, preferably by the control unit of the system. Therefore, sensor units can be used to gather such patient parameter for evaluation of the respective state of condition of the patient. Moreover, even additional information concerning the surrounding environment of the patient, like the time of the day, weather information, intended activities of the day or the like, can be taken into consideration for an adaption of the therapy.

Furthermore, the recording electrode of the electrode device may also provide input, in terms of current and/or voltage signals being determined by the at least one recording electrode in vicinity of the stimulation electrode of the electrode device. Thus, also any parameter concerning the interaction of the system with the patient's tissue can be considered for adaption of the stimulation, like a degeneration of the electrode device, in particular of its stimulation electrode, a degeneration of the surrounding tissue and/or an encapsulation by the surrounding tissue.

The system is configured to be used in the context of neuromodulation application. In the sense of the present invention neuromodulation may refer to recording of neural signals/acitivities within/around the nervous tissue, stimulation of nervous/neural tissue or a combination thereof.

The system according to the present invention can particularly be configured for applications like Peripheral Nerve Stimulation (PNS), Central Nerve Stimulation (CNS), Vagus Nerve Stimulation (VNS), Deep Brain Stimulation/Modulation (DBS/DBM), Spinal Cord Stimulation (SCS), Transcutaneous Electrical Nerve Stimulation (TENS) (e.g. for pain relief, tremor reduction, stroke recovery or athletic performance) or the like.

Preferably, such exemplary applications can be provided and executed in terms of closed-loop applications.

In accordance with the respective field of application, i.e. with the specific treatment application, the system can be provided as a completely external system or a partially implanted system, i.e. partially implanted in the patient's body.

Particularly, the electrode device, the control unit and/or the sensor unit can be provided as implantable or non-implantable components of the system, respectively.

In case of being provided as non-implantable components of the system, an arrangement along the patient's body, particularly along the patient's skin, as well as an integration into the patient's clothing, attachment means like (velcro) belts, smartwatches, fitness tracking devices or the like is possible.

The at least one sensor unit is configured to determine sensor data concerning the patient, and preferably vital and/or non-vital parameters of the patient. Such sensor data can also concern the environment of the patient, the respective circumstances referring to e.g. the daily activity of the patient or even further health-related information.

In the context of the present invention, vital and/or non-vital parameters of the patient can refer to e.g. blood pressure, hemodynamic pressure, glucose level, heart rate, oxygen level/tissue oxygenation or the detection of motions, positions, angles, etc. of the patient and/or his extremities.

Hence, one sensor unit can be provided in form of an inertial measurement unit which allows for detection of such motions, positions, angles and the like of the patient and his extremities.

Moreover, the control unit is configured to control and/or regulate the system. The information about the patient and the respective treatment can be provided to the control unit and processed by the control unit accordingly.

In this context, the control unit may also be configured to automatically adapt the respective treatment accordingly, at least within certain boundaries of treatment parameters, or at least to give advice for treatment parameter adjustment to the patient or a user of the system, i.e. the medical staff like e.g. a physician/doctor.

In summary, the control unit preferably captures all relevant information and is capable of providing individual adaption of the patient's treatment in the respective field of application. Hence, the system can preferably provide a closed-loop procedure.

Alternatively or in addition thereto, the system can also be configured to operate, at least temporarily, in form of an open-loop procedure, in particular in case of a sensor failure or the like. Hence, the system can continue its neuromodulation operation, e.g. on basis of former data and/or estimations/predictions concerning the development of the patient's health status/condition.

According to one embodiment of the present invention the at least one sensor unit comprises at least one sensor device which is provided as an implantable sensor device or as an external sensor device.

In particular, the external sensor device is considered to represent a non-implantable sensor device. Hence, the sensor device being provided in an external configuration can be embedded in smart clothing for example or provided as a patch, a smartwatch or the like, in order to be preferably arranged in vicinity of the patient and/or along the patient's body.

Hence, a comfortable and easy use of the system by the patient can be provided, whereby also an exchange of defective sensor devices can preferably be done by the patient himself.

In another preferred embodiment the at least one sensor unit comprises a plurality of sensor devices, wherein at least multiple of the plurality of sensor devices are provided as implantable sensor devices or at least multiple of a plurality of sensor devices are provided as external sensor devices.

In case multiple sensor devices are provided as a sensor unit, different kinds of sensor devices, namely implantable or external/non-implantable sensor devices, can be used.

In particular, sensor devices with rather short lifetime cycles, due to the sensor configuration itself, the energy consumption or due to e.g. hygienic aspects, may be provided as external ones in order to allows for an easy exchange on a regular basis within certain time ranges.

Thus, the system provides complete flexibility with regard to the sensor unit and the corresponding sensor devices.

In one embodiment at least one external sensor device is provided as a smart wear device, in particular in form of a skin patch, a smartwatch, sports or smart cloth, health electrodes or the like, being configured to provide vital and/or non-vital parameter monitoring such as a heart rate, tissue oxygenation, blood pressure, skin and/or body hydration, skin and/or body impedance and/or body composition, in particular body ratios concerning fat, muscle, water and the like.

Hence, smart devices like watches or smart clothing can ease the usage of the system in daily life for the patient. Moreover, by using such non-invasive techniques, also hygienic and comfort aspects can be improved.

According to another preferred embodiment of the present invention, the system further comprises at least one nerve sensor device, in particular an implantable nerve sensor device, e.g. a nerve cuff sensor or the like, which is (data) communicatively connected with the control unit.

In the context of the present invention, such nerve sensor device can particularly be considered as a satellite nerve sensor of the electrode device.

The nerve sensor device particularly allows for monitoring of nerve activity at a location being remote from the electrode device itself.

In consequence, the nerve sensor device allows for an individual monitoring and surveillance of the patient, particularly dependent on the respective treatment application and the parameters to be monitored in the respective context.

In one further embodiment the electrode device is an implantable electrode device or an external electrode device, in particular being configured for arrangement on the patient's skin.

Particularly depending on the respective field of application, an easy to use concept of the system can be provided by electrode devices being configured as external electrode devices, for example embedded in smart devices, smart clothes, attachment means etc.

In case the respective field of application requires the necessity to gather data by invasive measurement and/or to provide invasive stimulation to the patient's tissue, the electrode device can also be provided as an implantable electrode device.

The system according to the present invention provides full flexibility to be utilized in a wide range of different fields of application.

According to another embodiment the system further comprises an external communication unit, in particular configured to provide data transfer means and/or communication means and/or input means to the control unit, being preferably arranged along the patient's body.

The external communication unit can ensure safe and reliable communication with the control unit, in particular in case the control unit is provided in form of an implanted control unit. The external communication unit can for example provide an encrypted communication with the associated control unit.

In this context, the external communication unit can receive information and/or configuration commands from e.g. a user and forward them to the control unit. Moreover, the external communication unit can also provide a data storage means or the like, for the control unit.

By the external communication unit a communication with the control unit in the near field of the patient can be provided in a reliable, secure and energy saving manner.

In another embodiment the system comprises at least one external user interface device which is configured to provide a user interface and/or at least one sensor unit.

Via the user interface device the patient and/or a user like medical staff can gather information from the system and/or adjust parameters, for example treatment parameters or boundaries of treatment parameters within which the control unit can adapt the respective treatment.

The patient or user can observe the respective patient parameters, as measured and provided by the system, and/or provide additional input if considered necessary.

In this context, also a dashboard can be provided for the patient/user by the user interface, preferably illustrating the most relevant parameters or warnings at the point in time.

Such external user interface device can be provided in form of a smartwatch, a handheld device, a smartphone, a tablet, a (personal) computer or the like.

Moreover, such external user interface device can also provide and include an additional sensor unit. For example, a smartwatch can comprises sensor means for measurement of the heart rate, the blood oxygen level, parameters concerning an ECG or the like.

In another preferred embodiment, the at least one (external) user interface device comprises user input means and/or user output means .

Thus, the patient and/or a user, like a doctor/physician or other medical staff, can observe the patient's status as determined and monitored by the system as well as provide input, e.g. for the purpose of adapting the configuration of the treatment parameters.

The user input means can be provided in form of a touchscreen of e.g. a smartphone or a tablet or in the form of usual input means of (personal) computers.

The output can be provided in form of acoustic, visual or haptical output. Hence, the output means can be for example a display, like the display of a tablet, smartphone, smartwatch or the like, and/or a corresponding speaker. Preferably, an overview of the patient's status and the respective treatment status can be visualized, e.g. in form of a dashboard overview or the like.

The at least user interface device preferably provides for an easy access to information and configuration of the system by the patient and/or a user.

According to another embodiment the system further comprises a server in (data) communicative connection with the control unit and/or the at least one user interface device.

In particular, the server can provide a data cloud for storing and/or forwarding (information/configuration) data, e.g. between the control unit and the external user interface device.

It is also possible that a physician/doctor can provide adapted treatment configurations via input means at the server which are subsequently forwarded to the control unit directly or via the external communication unit.

Moreover, the server can also provide a data storage or further data/signal processing means in order to elaborate/calculate optimum treatment parameters for the respective patient, instead of or in additional support of the control unit.

According to a further embodiment, the components of the system are connected with each other by a wired connection, in particular an electrical or an optical connection, and/or by a wireless connection, in particular an inductive, a radiofrequency and/or an ultrasound connection, in order to provide energy transfer and/or a (data) communicative connection.

Moreover, further commonly known techniques to provide energy and/or data transfer, like wireless lan or bluetooth connections, can be used in the context of the present invention.

**In** the context of the present invention a (data) communicative connection is particularly configured to allow an unidirectional or bidirectional exchange of data between two entities/components of the system.

An inductive connection can particularly be provided in terms of a magnetic inductive field, hence by the effect of magnetic induction. Moreover, radiofrequency connections can be particularly provided in form of high frequency fields as well as ultrasound connections can be particularly implemented in form of ultrasound waves.

Depending from the respective purpose of the connection between the different components of the system, like the control unit, the electrode device, the sensor unit, the server, the external user interface device etc., an appropriate wired or unwired connection can be provided.

For example, an energy transfer to the control unit can be provided via an electrical cable connection or via an inductive connection. **In** contrast thereto, a suitable connection between the external user interface device and the external communication unit can, for example, be provided as a radiofrequency connect, an optical connection or an electrical wire connection, in order to provide a (data) communicative connection.

Thus, the present invention provides a high flexibility/versatility on how to configure the respective system, also in consideration of the patient specific needs.

**In** one preferred embodiment of the invention, the electrode device has at least one electrode comprising graphene, in particular being made of graphene or a graphene-based material or comprising a graphene (based) coating.

Preferably, different forms of graphene (based) materials may be used in the context of the present invention, like e.g. reduced graphene oxide (rGO), graphene oxide, chemical vapour deposited graphene (CVD Graphene) or any other potential form of graphene.

**In** particular, such graphene based materials can provide improved electrical and mechanical properties, e.g. a beneficial flexibility of the resulting electrode.

Such graphene electrodes particularly provide higher safe charge injection capacity as well as the signal-to-noise ratio/performance can be improved. Thereby, the electrode size can be reduced, even if the same amount of electrodes is maintained.

Hence, along a cross-section of the electrode device the cross-sectional area of the at least one electrode can be reduced.

Moreover, such graphene-based electrodes can provide for a safe electrical interface in aqueous environments, like in the context of neuromodulation of nervous tissue.

In summary, the safety and efficiency of the at least one electrode can be improved by also providing enhanced mechanical and structural properties of the electrode.

In a further preferred embodiment of the present invention, the system is configured to be used in one of the following applications:
- Neural-controlled motor prostheses applications, in particular referring to bidirectional implants for the control of an artificial limb;
- Electrocardiography (ECG);
- Electroencephalography (EEG);
- Electromyography (EMG);
- Smart wear applications, in particular skin patches, smartwatches, sports or smart cloth, health electrodes or the like, for vital and/or non-vital monitoring such as heart rate, tissue oxygenation, blood pressure, skin/body hydration / impedance and/or body composition measurements (fat, muscles, water, etc.);
- Transcutaneous Electrical Nerve Stimulation (TENS) in order to provide pain relief, tremor reduction, stroke recovery, athletic performance or the like;
- Micro-recording electrodes/needles (MER) applications;
- Defibrillation applications;
- Radiofrequency ablation applications;
- Temporal Interference stimulation applications;
- Cochlear device applications; and/or
- Intravascular device applications.

In particular, the components of the system can be provided and configured dependent from the individual application and the patient specific needs.

For example, in case of an ECG application, electrodes of the electrode device can be provided in form of patches, in order to measure electrical signals from the heart and/or to apply defibrillation shock/stimulation pulses.

In another exemplary case, the neural-controlled motor prostheses application may necessitate the usage of bidirectional implants and/or may allow for a combination of bidirectional implants in combination with external electrode devices and/or sensor units.

In consequence, the system according to the present invention provides a high flexibility/versatility of the configuration which thus can be individually adapted to the specific field of application as well as the patient's situation and needs.

On this basis, the system can particularly provide a closed-loop treatment which allows for adaption and configuration of optimum treatment parameters in order to enhance the efficiency, the effect as well as the patient's comfort and the system's lifecycle performance.

Further details and advantages of the present invention shall now be disclosed in the connection with the enclosed drawing.

It is shown in:

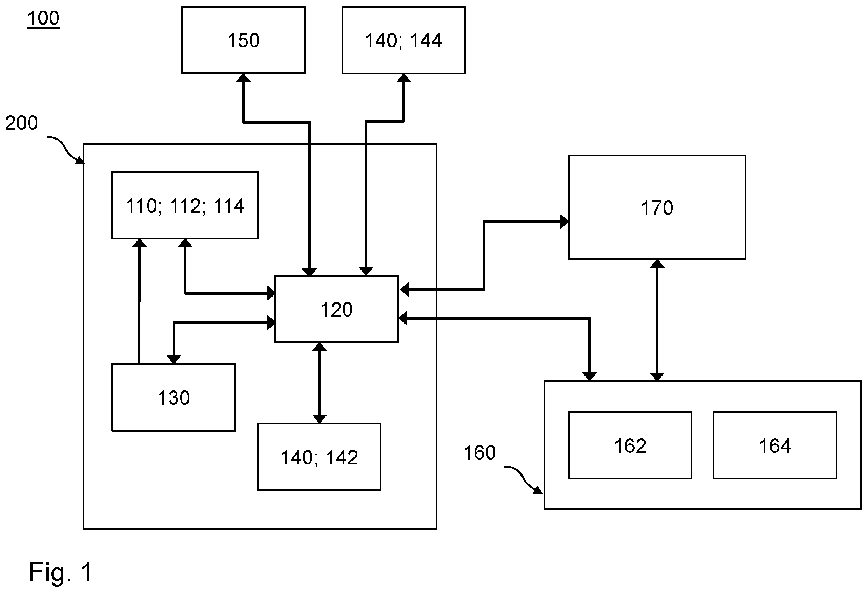

- **Fig. 1**: a schematic illustration of an embodiment of a system.

According to Fig. 1 a system 100 is shown with multiple components, whereby some of the components are implanted in a patient 200.

Implanted in the patient 200 the system 100 comprises an electrode device 110, a control unit 120, a nerve sensor device 130 and a sensor unit 140, at least an implanted sensor device 142 of the sensor unit 140.

The electrode device 110 is provided with a stimulation electrode 114 and a recording electrode 114.

The electrodes 112; 114 can preferably comprise graphene, e.g. can be made of a graphene-based material or can comprise a graphene-based film/coating.

The nerve sensor device 130 can preferably be provided in form of a nerve cuff sensor or the like.

The system 100 according to Fig. 1 is further provided with an external communication unit 150, an external sensor device 144 of the sensor unit 140, a user interface device 160 and a server 170.

Preferably, the external sensor device 144 and/or the external communication unit can be arranged in proximity to the patient 200, e.g. along the patient's 200 body and in particular along the patient's 200 skin.

The external user interface device 160 is shown in Fig. 1 with a user input means 162 and a user output means 164.

Preferably, all of these components of the system 100 provide connections, in particular (data) communicative connections, with the control unit 120.

(Data) communicative connections are particularly configured to allow for transfer and exchange of data in general, thus allowing for communication between the different components.

Moreover, connections between the components of the system 100 can also be configured to provide energy transfer.

Dependent on the purpose of the respective connection, the components can be connected by wire or provide a wireless connection.

A wired connection can be implemented e.g. in form of an electrical wire or an optical wire.

A wireless connection can be implemented for example by magnetic induction (inductive connection), by ultrasound waves (ultrasound connection) or by radiofrequency fields (radiofrequency/high frequency connection).

The system 100 as described above in the context of Fig. 1 can particularly provide the functionality as described in the following.

The control unit 120 is in (data) communicative connection with all other components of the system 100 in order to provide and forward information and/or sensor data to the control unit 120.

On basis of information about the patient's condition, environment and/or circumstances, the control unit 120 can adapt the treatment in individual and patient specific manner.

A nerve and/or muscle stimulation can be initiated by the control unit by forwarding corresponding signals to the stimulation electrode 112 of the electrode device 110.

The recording electrode 114 of the electrode device 110 can capture, determine and record electrical signals from the patient's tissue, in particular in vicinity of the stimulation electrode 112.

The recording electrode 114 can provide information to the control unit 120 from the area of stimulation.

In addition, electrical signals particularly resulting from nerve and/or muscle stimulation can also be determined by the (satellite) nerve sensor device 130.

The nerve sensor device 130 can particularly determine electrical signals at locations of the patient's 200 body being remote form the electrode device 110.

Signals/data as captured by the nerve sensor device 130 can be forwarded to the control unit 120 directly or via the electrode device 110.

The system 100 further comprises an implanted sensor unit 140 with the at least one implanted sensor device 142.

Moreover, the sensor unit 140, or a second sensor unit 140, can be provided with an external sensor device 144.

The sensor devices 142; 144 can be configured to determine vital and/or non-vital parameters of the patient, like blood/tissue oxygenation, heart rate, glucose level, body composition with regard to fat, muscle and water, or comparable parameters.

In particular, the implanted sensor device 142 may be configured to determine patient's parameter which may be measurable at all, at least more reliably measurable, on the inside of the patient's 200 body.

The external sensor device 144 may be particularly used for measuring parameters like the heart rate, which can be reliably and sufficiently accurate determined from the outside of the patient's body.

The sensor data as determined and gathered by the sensor unit, i.e. by the external sensor device 144 and the implanted sensor device 142, are forwarded to the control unit 120 accordingly.

The connections between the control unit 120 and the different components can be unidirectional or bidirectional.

In particular, bidirectional connections can also be provided to the sensor units 142; 144 and/or the nerve sensor device 130 in order to allow the control unit 120 to monitor and handle the data acquisition.

The control unit 120 can be configured to actively request sensor data from e.g. the sensor unit 140 or the nerve sensor device 130 and/or adapt the respective sampling frequency.

The control unit 120 is further connected to the external communication unit 150, which is preferably arranged in direct vicinity of the patient's 200 body, preferably along the patient's 200 body.

The external communication unit 150 can provide a reliable and save connection to the control unit 120, in particular for components being located outside and/or remote from the patient 200.

The external communication unit 150 may particularly be configured to communicate with the control unit 120 in an encrypted manner, in order to ensure a secure operation of the system 100.

In this way, the external communication unit 150 can forward data to the control unit 120 being received from other components of the system 100 or can forward data to the other components of the system 100 being received from the control unit 120.

The control unit 120 is further (data) communicatively connected with the external user interface device 160 and the server 170.

The user interface device 160 can provide information to the patient 200 and/or a user, like medical staff, by displaying e.g. parameter values concerning the patient's condition.

For example, the user output means can be provided via a touchscreen display and/or in acoustic manner via speakers.

In order to enhance the output, the user interface device can preferably provide a dashboard overview, categorizing and displaying the most relevant parameters being captured and recorded by the system 100, i.e. by the control unit 120 and the associated components of the system 100.

The user output means 164 may particularly provide warning indications and/or warning messages to notify the patient 200 and/or a user of e.g. necessary drug intake and/or critical patient conditions.

The external user interface device 160 can further comprise user input means 164 for providing input by the patient 200 and/or a user.

In particular, a patient 200 or a user can provide input to medications being taken or modify treatment parameters ranges within which the control unit 120 is allowed to modify the treatment parameter on its own, particularly without further approval by a medical staff.

The user input means 162 can further allow for additional data requests or data analysis by the patient 200 and/or a user, for example to generate predictions on treatment parameter or the patient's condition.

The user interface device 160 and/or the control unit 120 can be connected to the server 170.

The server 170 can particularly provide a data storage cloud and/or a data processing cloud.

The server 170 can serve as a central data storage. The server 170 can further allow medical staff, like a physician/doctor, to provide input from a remote location.

The input from the server 170 can be directed to the control unit 120 of the system 100 directly, or alternatively be forwarded via the external communication device 150.

The control unit 120 can adapt the neuromodulation/treatment accordingly, in particular in an individual and patient specific manner.

In summary, the present invention provides a system 100 which is highly flexible/versatile with respect to its configuration(s) and improves the patient's comfort as well as it can provide optimized treatment for a wide range of various applications concerning the field of neuromodulation.

In particular, the present invention preferably provides a closed-loop system 100, whereby the control unit 120 is capable of controlling and/or regulating the treatment (parameters). Therefore, information, e.g. in form of sensor data as captured and determined by the sensor unit 140, are provided to the control unit 120 for analysis and processing in order to optimized the treatment parameter configuration.

Hence, the patient's daily comfort, due to optimized treatment conditions, as well as the system's energy consumption and lifecycle performance can be improved.

Moreover, by the option to provide the different components of the system 100 as implanted or external components, e.g. the electrode device 110, a beneficial configuration, in view of costs, hygienic circumstances, exchange of defective parts in terms of maintenance, patient comfort, etc. can be achieved.

### Reference numerals

- 100: Neuromodulation system
- 110: Electrode device
- 112: Stimulation electrode
- 114: Recording electrode
- 120: Control unit
- 130: (Satellite) nerve sensor device
- 140: Sensor unit
- 142: Implantable sensor device
- 144: External sensor device
- 150: External communication unit
- 160: User interface device
- 162: User input means
- 164: User output means
- 170: Server
- 200: Patient

## Claims

1. System (100) for neuromodulation applications, in particular for monitoring and/or adaption of neuromodulation, comprising:
- at least one electrode device (110) with at least one stimulation electrode (112) and at least one recording electrode (114),
- at least one control unit (120), and
- at least one sensor unit (140) for determining sensor data, in particular vital and/or non-vital parameters of the patient,
wherein the control unit (120) is configured to adapt and/or initiate a neuromodulation treatment by applying electrical pulses via the at least one stimulation electrode (112) on basis of current signals and/or voltage signals as determined by the at least one recording electrode (114) and on basis of sensor data as provided by the at least one sensor unit (140), and
wherein the electrode device (110) has at least one electrode (112; 114) ) made of reduced graphene oxide, rGO.

2. System (100) according to claim 1,
**characterized in that**
the at least one sensor unit (140) comprises at least one sensor device (142; 144) which is provided as an implantable sensor device (142) or as an external sensor device (144).

3. System (100) according to claim 1 or 2,
**characterized in that**
the at least one sensor unit (140) comprises a plurality of sensor devices (142; 144),
wherein at least multiple of the plurality of sensor devices (142; 144) are provided as implantable sensor devices (142) or
at least multiple of a plurality of sensor devices (142; 144) are provided as external sensor devices (144).

4. System (100) according to claim 2 or 3,
**characterized in that**
at least one external sensor device (144) is provided as a smart wear device, in particular in form of a skin patch, a smartwatch, sports or smart cloth, health electrodes or the like, being configured to provide vital and/or non-vital parameter monitoring such as a heart rate, tissue oxygenation, blood pressure, skin and/or body hydration, skin and/or body impedance and/or body composition.

5. System (100) according to one of the preceding claims,
**characterized in that**
the system further comprises at least one nerve sensor device (130), in particular an implantable nerve sensor device (130), e.g. a nerve cuff sensor or the like, which is (data) communicatively connected with the control unit (120).

6. System (100) according to one of the preceding claims,
**characterized in that**
the electrode device (110) is an implantable electrode device (110) or an external electrode device (110), in particular being configured for arrangement on the patient's skin.

7. System (100) according to one of the preceding claims,
**characterized in that**
the system further comprises an external communication unit (150), in particular configured to provide data transfer means and/or communication means and/or input means to the control unit (120), being preferably arranged along the patient's body.

8. System (100) according to one of the preceding claims,
**characterized in that**
the system (100) comprises at least one external user interface device (160) which is configured to provide a user interface and/or at least one sensor unit.

9. System (100) according to claim 8,
**characterized in that**
the at least one user interface device (160) comprises user input means (162) and/or user output means (164).

10. System (100) according to one of the preceding claims,
**characterized in that**
the system (100) further comprises a server (170) in (data) communicative connection with the control unit (120) and/or the at least one user interface device (160).

11. System (100) according to one of the preceding claims,
**characterized in that**
the components of the system (100) are connected with each other by a wired connection, in particular an electrical or an optical connection, and/or by a wireless connection, in particular an inductive, a radiofrequency and/or an ultrasound connection, in order to provide energy transfer and/or a (data) communicative connection.

12. System (100) according to one of the preceding claims,
**characterized in that**
the system (100) is configured to be used in one of the following applications:
- Neural-controlled motor prostheses applications, in particular referring to bidirectional implants for the control of an artificial limb;
- Electrocardiography (ECG);
- Electroencephalography (EEG);
- Electromyography (EMG);
- Smart wear applications, in particular skin patches, smartwatches, sports or smart cloth, health electrodes or the like, for vital and/or non-vital monitoring such as heart rate, tissue oxygenation, blood pressure, skin/body hydration / impedance and/or body composition measurements (fat, muscles, water, etc.);
- Transcutaneous Electrical Nerve Stimulation (TENS) in order to provide pain relief, tremor reduction, stroke recovery, athletic performance or the like;
- Micro-recording electrodes/needles (MER) applications;
- Defibrillation applications;
- Radiofrequency ablation applications;
- Temporal Interference stimulation applications;
- Cochlear device applications; and/or
- Intravascular device applications.

## Patentansprüche

1. System (100) für Neuromodulationsanwendungen, insbesondere zum Überwachen und/oder Anpassen einer Neuromodulation, umfassend:
- mindestens eine Elektrodenvorrichtung (110) mit mindestens einer Stimulationselektrode (112) und mindestens einer Aufzeichnungselektrode (114),
- mindestens eine Steuereinheit (120), und
- mindestens eine Sensoreinheit (140) zum Ermitteln von Sensordaten, insbesondere von Vitalparametern und/oder Nicht-Vitalparametern des Patienten,
wobei die Steuereinheit (120) dazu ausgebildet ist, eine Neuromodulationsbehandlung durch Anlegen elektrischer Pulse über die mindestens eine Stimulationselektrode (112) auf der Grundlage von Stromsignalen und/oder Spannungssignalen, wie sie durch die mindestens eine Aufzeichnungselektrode (114) ermittelt werden, und auf der Grundlage von Sensordaten, wie sie durch die mindestens eine Sensoreinheit (140) bereitgestellt werden, anzupassen und/oder einzuleiten, und
wobei die Elektrodenvorrichtung (110) mindestens eine aus reduziertem Graphenoxid (rGO) hergestellte Elektrode (112; 114) aufweist.

2. System (100) nach Anspruch 1,
**dadurch gekennzeichnet, dass**
die mindestens eine Sensoreinheit (140) mindestens eine Sensorvorrichtung (142; 144) umfasst, die als implantierbare Sensorvorrichtung (142) oder als externe Sensorvorrichtung (144) ausgebildet ist.

3. System (100) nach Anspruch 1 oder 2,
**dadurch gekennzeichnet, dass**
die mindestens eine Sensoreinheit (140) eine Mehrzahl von Sensorvorrichtungen (142; 144) umfasst,
wobei zumindest mehrere aus der Mehrzahl von Sensorvorrichtungen (142; 144) als implantierbare Sensorvorrichtungen (142) bereitgestellt sind, oder
zumindest mehrere aus der Mehrzahl von Sensorvorrichtungen (142; 144) als externe Sensorvorrichtungen (144) bereitgestellt sind.

4. System (100) nach Anspruch 2 oder 3,
**dadurch gekennzeichnet, dass**
mindestens eine externe Sensorvorrichtung (144) als Smart-Wear-Vorrichtung, insbesondere in Form eines Hautpflasters, einer Smartwatch, in Form von Sportbekleidung oder intelligenter Kleidung, von Gesundheitselektroden oder dergleichen ausgebildet ist, und dazu eingerichtet ist, eine Überwachung von Vitalparametern und/oder Nicht-Vitalparametern, wie Herzfrequenz, Gewebeoxygenierung, Blutdruck, Haut- und/oder Körperhydratation, Haut- und/oder Körperimpedanz und/oder Körperzusammensetzung, bereitzustellen.

5. System (100) nach einem der vorhergehenden Ansprüche,
**dadurch gekennzeichnet, dass**
das System ferner mindestens eine Nervensensorvorrichtung (130), insbesondere eine implantierbare Nervensensorvorrichtung (130), beispielsweise einen Nervenmanschettensensor oder dergleichen, umfasst, die mit der Steuereinheit (120) datenkommunikativ verbunden ist.

6. System (100) nach einem der vorhergehenden Ansprüche,
**dadurch gekennzeichnet, dass**
die Elektrodenvorrichtung (110) eine implantierbare Elektrodenvorrichtung (110) oder eine externe Elektrodenvorrichtung (110) ist, die insbesondere zur Anordnung auf der Haut des Patienten ausgebildet ist.

7. System (100) nach einem der vorhergehenden Ansprüche,
**dadurch gekennzeichnet, dass**
das System ferner eine externe Kommunikationseinheit (150) umfasst, die insbesondere dazu ausgebildet ist, ein Datenübertragungsmittel und/oder Kommunikationsmittel und/oder Eingabemittel für die Steuereinheit (120) bereitzustellen, wobei die Kommunikationseinheit vorzugsweise entlang des Körpers des Patienten angeordnet ist.

8. System (100) nach einem der vorhergehenden Ansprüche,
**dadurch gekennzeichnet, dass**
das System (100) mindestens eine externe Benutzerschnittstellenvorrichtung (160) umfasst, die dazu ausgebildet ist, eine Benutzerschnittstelle und/oder mindestens eine Sensoreinheit bereitzustellen.

9. System (100) nach Anspruch 8,
**dadurch gekennzeichnet, dass**
die mindestens eine Benutzerschnittstellenvorrichtung (160) ein Benutzereingabemittel (162) und/oder ein Benutzerausgabemittel (164) umfasst.

10. System (100) nach einem der vorhergehenden Ansprüche,
**dadurch gekennzeichnet, dass**
das System (100) ferner einen Server (170) umfasst, der mit der Steuereinheit (120) und/oder der mindestens einen Benutzerschnittstellenvorrichtung (160) datenkommunikativ verbunden ist.

11. System (100) nach einem der vorhergehenden Ansprüche,
**dadurch gekennzeichnet, dass**
die Komponenten des Systems (100) untereinander über eine drahtgebundene Verbindung, insbesondere eine elektrische oder eine optische Verbindung, und/oder über eine drahtlose Verbindung, insbesondere eine induktive, hochfrequente und/oder Ultraschallverbindung, verbunden sind, um eine Energieübertragung und/oder eine datenkommunikative Verbindung bereitzustellen.

12. System (100) nach einem der vorhergehenden Ansprüche,
**dadurch gekennzeichnet, dass**
das System (100) zur Verwendung in einer der folgenden Anwendungen ausgebildet ist:
- Anwendungen für neuronal gesteuerte Motorprothesen, insbesondere bezogen auf bidirektionale Implantate zur Steuerung von künstlichen Gliedmaßen;
- Elektrokardiographie (EKG);
- Elektroenzephalographie (EEG);
- Elektromyographie (EMG);
- Smart-Wear-Anwendungen, insbesondere Hautpflaster, Smartwatches, Sportbekleidung oder intelligente Kleidung, Gesundheitselektroden oder dergleichen, zur Überwachung von Vitalparametern und/oder Nicht-Vitalparametern, wie Herzfrequenz, Gewebeoxygenierung, Blutdruck, Haut-/Körperhydratation, Haut-/Körperimpedanz und/oder Messungen der Körperzusammensetzung (Fett, Muskeln, Wasser usw.);
- transkutane elektrische Nervenstimulation (TENS) zur Bereitstellung von Schmerzlinderung, Tremorreduktion, Schlaganfallrehabilitation, sportlicher Leistungsfähigkeit oder dergleichen;
- Anwendungen in Zusammenhang mit Mikroaufzeichnungselektroden/-nadeln (MER);
- Defibrillationsanwendungen;
- Anwendungen in Zusammenhang mit einer Hochfrequenzablation;
- Anwendungen in Zusammenhang mit einer Temporal-Interference-Stimulation;
- Anwendungen in Zusammenhang mit einer Cochlea-Vorrichtung; und/oder
- Anwendungen in Zusammenhang mit einer intravaskulären Vorrichtung.

## Revendications

1. Système (100) pour des applications de neuromodulation, en particulier pour la surveillance et/ou l'adaptation d'une neuromodulation, comprenant :
- au moins un dispositif d'électrode (110) comprenant au moins une électrode de stimulation (112) et au moins une électrode d'enregistrement (114),
- au moins une unité de commande (120), et
- au moins une unité de capteur (140) destinée à déterminer des données de capteur, en particulier des paramètres vitaux et/ou non vitaux du patient,
dans lequel l'unité de commande (120) est configurée pour adapter et/ou initier un traitement de neuromodulation par application d'impulsions électriques via ladite au moins une électrode de stimulation (112) sur la base de signaux de courant et/ou de signaux de tension déterminés par ladite au moins une électrode d'enregistrement (114) et sur la base de données de capteur fournies par ladite au moins une unité de capteur (140), et
dans lequel le dispositif d'électrode (110) comprend au moins une électrode (112; 114) fabriquée en oxyde de graphène réduit (rGO).

2. Le système (100) selon la revendication 1,
**caractérisé en ce que**
ladite au moins une unité de capteur (140) comprend au moins un dispositif de capteur (142; 144) configuré comme dispositif de capteur implantable (142) ou comme dispositif de capteur externe (144).

3. Le système (100) selon la revendication 1 ou 2,
**caractérisé en ce que**
ladite au moins une unité de capteur (140) comprend une pluralité de dispositifs de capteur (142; 144),
dans lequel au moins plusieurs de la pluralité de dispositifs de capteur (142; 144) sont prévus sous la forme de dispositifs de capteur implantables (142), ou
au moins plusieurs de la pluralité de dispositifs de capteur (142; 144) sont prévus sous la forme de dispositifs de capteur externes (144).

4. Le système (100) selon la revendication 2 ou 3,
**caractérisé en ce que**
au moins un dispositif de capteur externe (144) est configuré comme dispositif de type smart-wear, en particulier sous forme d'un patch cutané, d'une montre connectée, de vêtements de sport ou de vêtements intelligents, d'électrodes de santé ou similaires, et est configuré pour fournir une surveillance de paramètres vitaux et/ou non vitaux, tels que la fréquence cardiaque, l'oxygénation des tissus, la pression artérielle, l'hydratation cutanée et/ou corporelle, l'impédance cutanée et/ou corporelle et/ou la composition corporelle.

5. Le système (100) selon l'une des revendications précédentes,
**caractérisé en ce que**
le système comprend en outre au moins un dispositif de capteur nerveux (130), en particulier un dispositif de capteur nerveux implantable (130), par exemple un capteur de type manchette nerveuse ou similaire, qui est en communication de données avec l'unité de commande (120).

6. Le système (100) selon l'une des revendications précédentes,
**caractérisé en ce que**
le dispositif d'électrode (110) est un dispositif d'électrode implantable (110) ou un dispositif d'électrode externe (110), en particulier configuré pour être disposé sur la peau du patient.

7. Le système (100) selon l'une des revendications précédentes,
**caractérisé en ce que**
le système comprend en outre une unité de communication externe (150), en particulier configurée pour fournir un moyen de transmission de données et/ou un moyen de communication et/ou un moyen d'entrée pour l'unité de commande (120), ladite unité de communication étant de préférence disposée le long du corps du patient.

8. Le système (100) selon l'une des revendications précédentes,
**caractérisé en ce que**
le système (100) comprend au moins un dispositif d'interface utilisateur externe (160) configuré pour fournir une interface utilisateur et/ou au moins une unité de capteur.

9. Le système (100) selon la revendication 8,
**caractérisé en ce que**
ledit au moins un dispositif d'interface utilisateur (160) comprend un moyen d'entrée utilisateur (162) et/ou un moyen de sortie utilisateur (164).

10. Le système (100) selon l'une des revendications précédentes
**caractérisé en ce que**
le système (100) comprend en outre un serveur (170) en communication de données avec l'unité de commande (120) et/ou ledit au moins un dispositif d'interface utilisateur (160).

11. Le système (100) selon l'une des revendications précédentes,
**caractérisé en ce que**
les composants du système (100) sont connectés entre eux par une connexion filaire, en particulier une connexion électrique ou optique, et/ou par une connexion sans fil, en particulier une connexion inductive, à haute fréquence et/ou à ultrasons, afin de fournir un transfert d'énergie et/ou une connexion de communication de données.

12. Le système (100) selon l'une des revendications précédentes,
**caractérisé en ce que**
le système (100) est configuré pour être utilisé dans l'une des applications suivantes :
- des applications pour des prothèses motrices commandées par voie neuronale, en particulier relatives à des implants bidirectionnels pour la commande d'un membre artificiel ;
- électrocardiographie (ECG) ;
- électroencéphalographie (EEG) ;
- électromyographie (EMG) ;
- applications de type smart-wear, en particulier patchs cutanés, montres connectées, vêtements de sport ou vêtements intelligents, électrodes de santé ou similaires, pour la surveillance de paramètres vitaux et/ou non vitaux, tels que la fréquence cardiaque, l'oxygénation tissulaire, la pression artérielle, l'hydratation cutanée et/ou corporelle, l'impédance cutanée et/ou corporelle et/ou des mesures de composition corporelle (graisse, muscles, eau, etc.) ;
- stimulation électrique nerveuse transcutanée (TENS) pour fournir un soulagement de la douleur, une réduction des tremblements, une rééducation post-AVC, une amélioration des performances sportives ou similaires ;
- applications liées à des microélectrodes/aiguilles d'enregistrement (MER) ;
- applications de défibrillation ;
- applications liées à l'ablation par radiofréquence ;
- applications liées à la stimulation par interférence temporelle ;
- applications liées à un dispositif cochléaire ; et/ou
- applications liées à un dispositif intravasculaire.
